# FASCICULE DE BREVET EUROPEEN

(11) **EP 1 443 940 B1**
(45) Date de publication et mention de la délivrance du brevet: **20.09.2006**
(21) Numéro de dépôt: 02788051.7
(22) Date de dépôt: 13.11.2002
(51) Int. Cl.: A61K 31/57, A61K 9/48

(54) **COMPOSITION PHARMACEUTIQUE A BASE DE PROGESTERONE MICRONISEE, SON PROCEDE DE PREPARATION ET SES UTILISATIONS**
ARZNEIZUSAMMENSETZUNG BESTEHEND AUS MIKRONISIERTEM PROGESTERON, VERFAHREN ZU IHRER HERSTELLUNG UND IHRE VERWENDUNGEN
PHARMACEUTICAL COMPOSITION BASED ON MICRONIZED PROGESTERONE, PREPARATION METHOD AND USES THEREOF

(30) Priorité: 13.11.2001 FR 0114653
(43) Date de publication de la demande: 11.08.2004
(73) Titulaire: Besins International Belgique, 1620 Drogenbos (BE)
(72) Inventeur: BESINS, Antoine, B-1050 Bruxelles (BE); BESSE, Jérôme, F-33480 Listrc Medoc (FR)
(74) Mandataire: Nargolwalla, Cyra
(86) Numéro de dépôt international: PCT/FR2002/003879
(87) Numéro de publication internationale: WO 2003/041720

(56) Documents cités:
- EP-A- 0 598 337
- WO-A-97/37642
- WO-A-97/40823
- FR-A- 2 408 345
- US-A- 5 140 021
- DATABASE WPI Section Ch, Week 198606 Derwent Publications Ltd., London, GB; Class B01, AN 1986-038601 XP002207311 & JP 60 258110 A (DAIGO EIYO KAGAKU KK), 20 décembre 1985 (1985-12-20)

## Description

La présente invention concerne une composition pharmaceutique contenant de la progestérone micronisée, de la lécithine de soja, et au moins une huile sélectionnée dans le groupe constitué par l'huile de tournesol, d'olive, de sésame, de colza et d'amande. Elle concerne également des spécialités pharmaceutiques comprenant ladite composition pharmaceutique.

L'invention concerne en outre le procédé de fabrication de cette composition pharmaceutique ainsi que ses utilisations.

La progestérone est une hormone qui est synthétisée, chez la femme, essentiellement par l'ovaire lors de la phase post-ovulaire ou lutéale (plus précisément par les cellules du corps jaune) et, à un moindre degré, par les glandes surrénales et le placenta au cours de la deuxième partie de la grossesse. Une synthèse non endocrine de la progestérone, notamment au niveau des neurones, est également possible.

L'insuffisance de la sécrétion de la progestérone chez une femme a pour conséquence une perte de ses effets biologiques : effet progestatif, effet anti-androgène (action sur la peau) et anti-oestrogène (avec pour conséquence une hyperoestrogénie : bouffées de chaleur, difficultés psychogènes de type anxieux ou dépressif, prise de poids,...). Cette insuffisance en progestérone peut conduire à des gênes fonctionnelles et des manifestations cliniques diverses, en particulier:
- syndromes prémenstruels,
- irrégularités menstruelles par disovulation ou anovulation,
- mastopathies bénignes,
- périménopause et ménopause.

Mais l'administration par voie orale de la progestérone souffre d'un grave handicap en raison de la faible absorption intestinale et de l'intense métabolisme hépatique (faible demi-vie plasmatique) de cette hormone. Seules les voies vaginales, rectales et intramusculaires permettraient jusqu'à présent de maintenir pendant plusieurs heures consécutives la progestéronémie au niveau physiologique de la phase lutéale.

Les LABORATOIRES BESINS-ISCOVESCO ont déjà proposé une solution afin d'améliorer la qualité et l'intensité de l'absorption digestive de la progestéone naturelle, dans la demande de brevet FR 76 36007. En effet, ils ont développé une formulation de capsules molles contenant de la progestérone micronisée en suspension huileuse. L'effet synergique de la micronisation et la mise en oeuvre de molécules contenant des acides gras à longue chaîne a permis d'augmenter indiscutablement la biodisponibilité de la progestérone par voie orale. Cette formulation a connu un très grand succès au niveau mondial. Elle est vendue en France sous la marque UTROGESTAN®.

L'huile qui sert de base à la suspension huileuse dans l'UTROGESTAN® est l'huile d'arachide.

L'arachide (*Arachis hypogae,* peanut) est une légumineuse, plante annuelle touffue à fleurs jaunes de la famille des Papilionacées.

Depuis 15 ans, l'allergie à l'arachide est devenue un problème allergologique important.

Dutau et al. (La Presse Médicale, vol. 28, p. 1553) observent que la prévalence de l'allergie à l'arachide a été récemment estimée à 1,3% dans la population générale. L'utilisation accrue de l'arachide dans l'alimentation, très souvent sous forme masquée, explique peut être cette évolution.

Des sensibilisations précoces ont été décrites chez les nourrissons qui n'avaient jamais consommé de l'arachide sous forme conventionnelle, mais qui furent apparemment sensibilisés *in utero* ou *via* le lait maternel, par des laits maternisés ayant contenu des graisses végétales (huile d'arachide) ou par des préparations médicamenteuses en solution huileuse.

L'UTROGESTAN® est susceptible d'être prescrit dans de nombreux cas d'indications thérapeutiques, y compris en tant que supplément de la phase lutéale au cours des cycles de fécondation in vitro (FIV), et en cas de menace d'avortement ou de prévention d'avortement à répétition par insuffisance lutéale, jusqu'à la 12^{ème} semaine de grossesse. Il est donc possible, en théorie, qu'un foetus soit exposé à l'UTROGESTAN® *in utero*.

A ce jour, si les effets allergènes de l'arachide sont certains, il existe toujours une controverse sur la capacité de l'huile d'arachide d'engendrer des réactions allergéniques. On peut citer de nombreuses publications à ce sujet, dont : Taylor et al., J. Allergy Clin. Immunol., vol. 68, p. 372 (1981) ; Moneret-Vautrin et al., Pediatr. Allergy Immunol. vol. 5, p. 184 (1994) ; Sabbah et Lauret, Allergie et Immunologie, vol. 26, p. 380 (1994) ; de Montis et al., Arch. Pédiatr., vol. 2, p. 25 (1995)).

Compte tenu de ce fait, la Société Demanderesse s'est consacrée au développement d'une nouvelle composition pharmaceutique, en remplaçant l'huile d'arachide par d'autres huiles qui ne présentent pas de hauts risques d'allergénicité, tout en s'efforçant de conserver les avantages de l'ancienne formule.

Après de nombreux travaux et recherches, lors desquels plusieurs huiles végétales ont été testées, l'huile de tournesol, d'olive, de sésame, de colza et d'amande ont été retenues. En effet, l'utilisation de ces huiles permet d'écarter les risques de réactions allergiques tout en conservant l'ensemble des caractéristiques physico-chimiques et cinétiques de l'ancienne, formulation d'UTROGESTAN®, caractéristiques qui étaient à l'origine de son succès. Etant donné que le procédé de fabrication de l'ancienne formulation d'UTROGESTAN® à l'échelle industrielle comportait des étapes dont la mise en oeuvre est très délicate, il est du mérite de la Société Demanderesse d'avoir réussi à modifier la formulation sans augmenter les difficultés de production.

Dans le contexte de la présente invention, les huiles peuvent être raffinées ou non. Une huile raffinée est une huile qui est obtenue en partant de l'huile brute et qui a subi un ensemble d'opérations de raffinage. L'huile raffinée est une huile purifiée présentant un très faible taux d'impuretés et exempte notamment de protéines potentiellement allergisantes telles que le gluten.

Dans la composition pharmaceutique selon l'invention, la progestérone micronisée se trouve de préférence en suspension dans l'huile de tournesol, d'olive, de sésame, de colza, d'amande ou dans un mélange de certaines ou de toutes ces huiles.

La Société Demanderesse a connaissance du brevet US 5 140 021 au nom de GENESIS SYSTEMS CORPORATION (Maxson et al.) qui décrit une capsule molle contenant une progestérone micronisée en suspension dans une huile hautement insaturée. L'huile de tournesol figure parmi les huiles citées dans ce brevet. Cependant, les inventeurs de ce brevet US 5 140 021 ont pris le plus grand soin de se distinguer de la spécialité pharmaceutique UTROGESTAN®, c'est à dire la formulation développée et actuellement commercialisée par la Société Demanderesse, à base de l'huile d'arachide. Ainsi, la progestérone micronisée utilisée dans le brevet de GENESIS SYSTEMS est décrite comme possédant une granulométrie particulière et différente de celle utilisée dans UTROGESTAN®.

Le brevet US 5 140 021 ne décrit qu'une préparation à l'échelle du laboratoire des capsules de progestérone, et ne fournit aucun enseignement quant à la préparation de capsules à une échelle industrielle. De plus, les capsules de progestérone selon ce brevet américain ne contiennent pas de lécithine de soja, un élément essentiel de la composition pharmaceutique selon la présente invention. En effet la lécithine de soja joue le rôle d'agent de suspension des particules de progestérone dans l'huile de tournesol et de lubrifiant lors de l'encapsulation du contenu à l'échelle industrielle.

L'invention concerne donc une composition pharmaceutique comprenant de la progestérone micronisée, de la lécithine de soja, et au moins une huile sélectionnée dans le groupe constitué par l'huile de tournesol, d'olive, de sésame, de colza et d'amande.

Selon un mode avantageux de réalisation de la composition pharmaceutique selon l'invention, la progestérone micronisée se trouve en suspension dans l'huile ou dans un mélange de certaines ou de toutes ces huiles.

Dans le contexte de la présente invention, on entend par « progestérone micronisée » une progestérone dont au moins 80% des particules ont une granulométrie comprise entre 1 et 15 µm, de préférence 50% des particules ont une granulométrie entre 1 et 10 µm, et plus préférentiellement encore 25% des particules ont une granulométrie entre 1 et 5 µm, ces granulométries étant mesurées par granulomètre laser de type Malvern, par la procédure décrite dans les exemples de la présente demande de brevet.

Lors des études préalables au choix de l'huile, la Société Demanderesse a pu observer, de façon surprenante et inattendue, que la combinaison de la lécithine de soja avec les huiles sélectionnées selon l'invention était très intéressante puisqu'elle ne modifiait pas la granulométrie de la progestérone micronisée en suspension dans l'huile. De plus, aucune différence significative au niveau de la distribution granulométrique de la progestérone micronisée en suspension n'est apparue entre la composition à base d'huile d'arachide et celle contenant les huiles utilisées conformément à l'invention.

Quant aux autres huiles testées, la granulométrie n'était pas la même en présence ou en l'absence de la lécithine de soja. L'intérêt essentiel des huiles sélectionnées selon l'invention sur un plan physico-chimique comparativement à l'huile d'arachide est d'assurer à la fois :
- des solubilités à saturation de la progestérone micronisée comparables;
- des granulométries de la suspension comparables ;
- et des profils de dissolution in vitro comparables.

Or, granulométrie et solubilité à saturation influencent de manière significative la biodisponibilité *in vivo* de la progestérone.

Le choix de ces huiles permet donc de mieux maîtriser la répartition granulométrique de la progestérone micronisée dans la suspension huileuse ainsi que le taux de progestérone solubilisée dans l'huile et donc de rassembler l'ensemble des conditions nécessaires au maintien d'une biodisponiblité in vivo similaire à celle obtenue avec UTROGESTAN®.

Selon un mode avantageux de réalisation de la composition pharmaceutique selon l'invention, le rapport progestérone/huile(s) est compris entre 0,15/1 et 3/1, de préférence entre 0,25/1 et 2/1, préférentiellement entre 0,40/1 et 1/1, et plus préférentiellement encore est de 0,67/1.

Selon un mode avantageux de réalisation de la composition pharmaceutique l'invention, le rapport lécithine de soja/huile(s) est compris entre 0,005/1 et 0,3/1, de préférence entre 0,01/1 et 0,2/1, préférentiellement entre 0,040/1 et 0,1/1, et plus préférentiellement encore est de 0,067/1.

La composition pharmaceutique selon l'invention peut également comprendre un oestrogène ou un de ses dérivés de type ester, de préférence sélectionné dans le groupe constitué par le 17-β estradiol, l'estrone, le 17α-éthinyl-estradiol, le valérate d'estradiol, ou les phyto-oestrogènes, et plus préférentiellement encore est le 17-β estradiol.

La composition pharmaceutique selon l'invention peut se présenter, entre autres, sous la forme d'une capsule molle, d'une gélule, d'un comprimé, ou d'une suspension buvable.

Lorsque la composition pharmaceutique selon l'invention est intégrée dans une spécialité pharmaceutique, chaque unité posologigue comprend avantageusement entre 2 mg et 600 mg de progestérone micronisée, de préférence entre 30 mg et 300 mg, et plus préférentiellement encore entre 100 mg et 200 mg.

La composition pharmaceutique selon l'invention peut être administrée par voie orale ou par voie vaginale selon les indications thérapeutiques.

L'administration par voie vaginale représente également une alternative à la voie orale en cas d'effets secondaires dus à la progestérone (somnolence après absorption par voie orale) ou de contre-indication à la voie orale (hépatopathie).

Selon un mode avantageux de réalisation de la composition pharmaceutique selon l'invention, la capsule comprend de la gélatine ou équivalent.

L'invention concerne également un procédé pour la préparation d'une composition pharmaceutique comprenant de la progestérone micronisée, de la lécithine de soja, et au moins une huile sélectionnée dans le groupe constitué par l'huile de tournesol, d'olive, de sésame, de colza et d'amande.

Ce procédé comporte les étapes successives suivantes :
- on réalise, sous agitation, un mélange d'huile(s) et de lécithine de soja afin d'obtenir un mélange ;
- on ajoute, sous agitation, la progestérone micronisée au mélange ainsi obtenu afin d'obtenir une suspension homogène.

Cette suspension peut être administrée telle que, sous forme d'une suspension buvable, ou être présentée sous la forme de capsules molles ou de gélules, mais peut également servir à l'imprégnation d'un support absorbant présenté sous la forme de poudre.

Ce support absorbant peut être du type maltodextrine et/ou dérivés, silice et/ou dérivés, cyclodextrine et/ou dérivés, poudre de cellulose et/ou dérivés ou leur association ou tout autre matière première pharmaceutique intégrant ces propriétés.

La poudre ainsi obtenue peut être présentée sous la forme de gélules ou de comprimés. Les gélules ou comprimés contenant la poudre peuvent également comprendre des agents liants, des agents désintégrants, des agents diluants et/ou des agents lubrifiants.

L'invention concerne également l'utilisation de la progestérone micronisée, de la lécithine de soja, et au moins une huile sélectionnée dans le groupe constitué par l'huile de tournesol, d'olive, de sésame, de colza et d'amande, pour la préparation d'un médicament pour le traitement d'une condition physiologique liée à l'insuffisance de la sécrétion de la progestérone.

On peut citer, comme exemples de telles conditions physiologiques : l'insuffisance lutéale, l'irrégularité menstruelle, les syndromes prémenstruels, les mastodynies, les mastopathies bénignes, la préménopause, la stérilité par insuffisance lutéale, les troubles de la ménopause, la contraception locale, la prévention d'avortements habituels en cas d'insuffisance lutéale, les menaces d'accouchement prématuré, l'acné, l'alopécie, la prévention de l'ostéoporose, les cancers de l'endomètre, et l'épilepsie.

L'invention concerne également l'utilisation de la progestérone micronisée, de la lécithine de soja, et au moins une huile sélectionnée dans le groupe constitué par l'huile de tournesol, d'olive, de sésame, de colza et d'amande, ainsi qu'un oestrogène, pour la préparation d'un médicament pour le traitement d'une condition physiologique liée à l'insuffisance de la sécrétion de la progestérone. L'oestrogène ou un de ses dérivés de type ester est de préférence sélectionné dans le groupe constitué par le 17-β estradiol, l'estrone, le 17α-éthinyl-estradiol, le valérate d'estradiol, ou les phyto-oestrogènes, et plus préférentiellement encore est le 17-β estradiol.

L'invention sera mieux comprise à l'aide des exemples décrits ci-dessous.

### EXEMPLE 1 : COMPOSITION PHARMACEUTIQUE SOUS LA FORME D'UNE CAPSULE MOLLE SELON L'INVENTION

Le contenu d'une capsule molle selon l'invention est décrite dans le tableau I ci-dessous.

**TABLEAU I**

| **Nom du composé** | **Formule Centésimale(%) Par unité(mg)** | | **Fonction** | **Références aux normes** |
|---|---|---|---|---|
| *Substance active* | | | | |
| • Progestérone micronisée | 40,00 | 100,00 | Substance Active | Ph.Eur.3^{ème} ed. |
| | | | | |
| *Excipients* | | | | |
| • Huile ou mélange d'huiles selon l'invention | 59,60 | 149,00 | Diluant | Ph.Eur.3^{ème} ed. |
| | | | | |
| • Lécithine de soja | 0,40 | 1,00 | Emulsifiant | USP 24, NF 19, p.2471. |

La Société Demanderesse a également préparé des capsules de 500 mg, qui sont homothétiques avec les capsules de 250 mg décrites ci-dessus. Ainsi, les capsules de 500 mg contiennent 200 mg de progestérone micronisée, 2 mg de lécithine de soja, et à titre d'exemple, 298 mg d'huile de tournesol.

### EXEMPLE 2 : ETUDE DE SOLUBILITE DE PROGESTERONE MICRONISEE DANS DIFFERENTES HUILES

Afin de sélectionner le véhicule huileux optimal pour remplacer l'huile d'arachide, tout en conservant les propriétés physico-chimiques de l'ancienne formulation, les huiles végétales suivantes ont été testées en ce qui concerne la solubilité de la progestérone dans ces huiles:
- huile d'arachide
- huile d'olive
- huile de tournesol
- huile de tournesol à haute teneur en acide oléique
- huile de colza
- huile d'amande
- huile de soja
- huile de sésame
- huile de maïs.

Des solutions standards ont été préparées comme suit :
- solution concentrée : Progestérone lot A0098 10mg
   Huile qsp 20ml
- solution diluée : solution concentrée 1 ml
   tetrahydrofuranne (THF) 10ml
   acétonitrile qsp 20ml
agitation magnétique : 5 minutes

Les solutions saturées ont ensuite été préparées comme suit :

Les solutions saturées dans chaque huile ont été maintenues pendant une heure sous agitation à température ambiante, puis ont été filtrées sur un seringue filtre de diamètre 25mm nylon à 0,45 µm.

Les solutions saturées ont été diluées à 1/200 :
- solution saturée : 0,5 ml
- THF : 50 ml
- acétonitrile qsp 100ml

Les résultats (concentration à saturation en progestérone en fonction des huiles testées) sont groupés dans les tableaux II et III ci-dessous :

**TABLEAU II**

| **HUILE TESTEE** | **CONCENTRATION A SATURATION (mg/ml)** | **ECART RELATIF* SUR LA CONCENTRATION A SATURATION (%)** |
|---|---|---|
| Huile d'arachide | 16,77 | - |
| Huile de colza | 18,14 | + 8.2% |
| Huile de tournesol | 17,50 | + 4.4% |
| Huile de tournesol à haute teneur en acide oléique | 8,29 | - 50.6% |
| Huile d'olive | 17,46 | + 4.1% |

**TABLEAU III**

| **HUILE TESTEE** | **CONCENTRATION A SATURATION (mg/ml)** | **ECART RELATIF* SUR LA CONCENTRATION A SATURATION (%)** |
|---|---|---|
| Huile d'arachide | 18,80 | - |
| Huile d'amande | 18,98 | + 1.0% |
| Huile de soja | 16,19 | - 13.9% |
| Huile de sésame | 19,80 | + 5.3% |
| Huile de maïs | 15,60 | - 17.0% |

| | | |
|---|---|---|
| * Huile d'arachide = huile de référence | | |

Les huiles de colza, de tournesol, d'olive, de sésame, et d'amande ont été retenues suite à cette étude de solubilité à saturation.

Parmi les différents fournisseurs d'huiles nous pouvons citer à titre d'exemple :
- pour l'huile d'olive : LESSIEUR ;
- pour l'huile de tournesol : HENRY LAMOTTE.

### EXEMPLE 3 : FABRICATION DE CAPSULES MOLLES DE PROGESTERONE MICRONISEE SELON L'INVENTION

La fabrication des capsules molles à base de progestérone micronisée selon l'invention se fait comme suit :

On préparé les capsules selon l'une des méthodes connues en soi à l'homme du métier.

Pour un lot de 2 300 000 capsules, chacune contenant 100 mg de progestérone, on procède de la manière suivante :

L'atmosphère est contrôlé à 22°C ± 3°C et à une humidité relative de 35% ± 10 %.

Les ingrédients suivants sont pesés:

| | |
|---|---|
| Progestérone | 230,00 Kg |
| Huile de tournesol | 342,70 Kg |
| Lécithine de soja | 2,30 Kg |

Un mélangeur de capacité de 600 litres est mis sous vide.

On introduit sous vide dans ce mélangeur 3/4 de la quantité d'huile de tournesol est ajouté la lécithine de soja.

On remet le mélangeur sous vide (entre 0.7 bars et 0.9 bars) et puis on agite à faible vitesse entre 10 rotations par minute et 15 rotations par minute.

On rajoute la progestérone sous vide et puis on rajoute le quart d'huile de tournesol restante et on amène la température à 23°C ± 3°C.

Ensuite, une agitation forte est effectuée jusqu'à homogénéisation.

Le mélangeur sous forte agitation est mis sous pression jusqu'à un maximum de 1 bar.

Un tamisage continu à l'aide d'un tamis de 500 µm est effectué et le mélange est transféré dans un récipient de stockage.

Les récipients de stockage sont mis sous vide et puis agités entre 2400 et 2000 rotations par minute pendant 15 minutes. Ils sont remis sous vide et agités à nouveau pendant 30 minutes à une vitesse entre 2000 rotations par minute et 2500 rotations par minute.

L'agitation est arrêtée et les récipients sont laissés pendant 5 minutes sous vide.

L'encapsulation est effectuée de manière classique, connue à l'homme du métier.

### EXEMPLE 4 : DETERMINATION DE LA GRANULOMETRIE D'UNE CAPSULE SELON L'INVENTION

Une étude granulométrique comparative a été effectuée entre des capsules d'UTROGESTRAN© et des capsules selon l'invention contenant de l'huile de tournesol.

Le matériel utilisé est le suivant :
- Mastersizer 2000 granulomètre laser
- Cellule de mesure Hydro 2000 SM

Selon la méthode suivante :
■ Quantité d'échantillon par mesure : 1 ou 2 gouttes déposées à la pipette
■ Milieu : Huile de tournesol saturée filtrée. Cette huile est préparée sous agitation magnétique en maintenant la température à 37°C, pendant 1 heure, puis filtrée sur filtre papier.
   - Indice de réfraction : (oil average) 1,4671
   - Volume de milieu : 100 ml
■ Vitesse d'agitation : 1800 rpm
■ Pourcentage d'obscuration : entre 10 et 20 %
■ Pourcentage résiduel pondéré : < 3 %

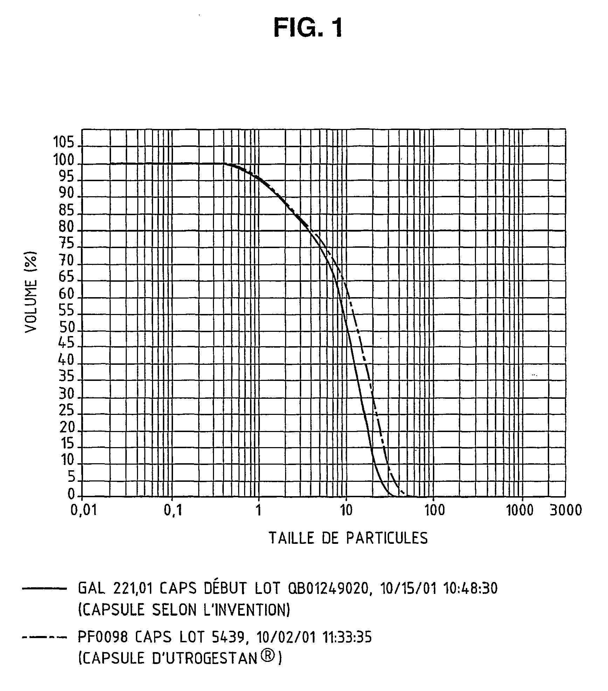

- Nombre de mesures par préparation : 2. Les mesures débutent après 30 min de stabilisation du pourcentage d'obscuration.Les résultats reportés dans la figure 1 ci-après démontrent que les granulométries des deux capsules sont fortement comparables.

D'autres études effectuées par la Société Demanderesse ont démontré que la distribution granulométrique de la progestérone dans l'huile de sésame, d'olive, de colza ou d'amande est également comparable avec celle obtenue dans l'huile d'arachide.

### EXEMPLE 5: ETUDE DE DISSOLUTION IN VITRO COMPARATIVE ENTRE UNE CAPSULE D'UTROGESTRAN© ET UNE CAPSULE SELON L'INVENTION.

On a utilisé un appareil de dissolution à paniers tournants SOTAX AT7.

Dans une fiole jaugée de 200 ml (classe A), on dissout 20 mg de progestérone exactement pesés dans 2 ml d'éthanol, puis on passe aux ultrasons et on complète au trait de jauge à l'aide du milieu de dissolution (Kleptose® 1 %).

La solution témoin est filtrée sur filtre seringue fibre de verre de porosité 1 µm.

On place 7 cuves dans un bain-marie à température constante, puis on transfère 1000 ml de milieu de dissolution dans chacune des 7 cuves.

On place 1 capsule dans 6 cuves, puis on immerge les paniers dans le milieu de dissolution à une distance de 25 mm ± 2 mm entre le panier et le fond de la cuve.

On met les paniers sous agitation, puis on prépare une solution témoin.

A chaque intervalle de temps prévu (5, 10, 15, 30, 45, 60, 90, 120, 150, 180, 225, 270, 315 et 360 minutes), les échantillons sont collectés puis analysés par Spectrophotométrie UV (λ: 248 nm).

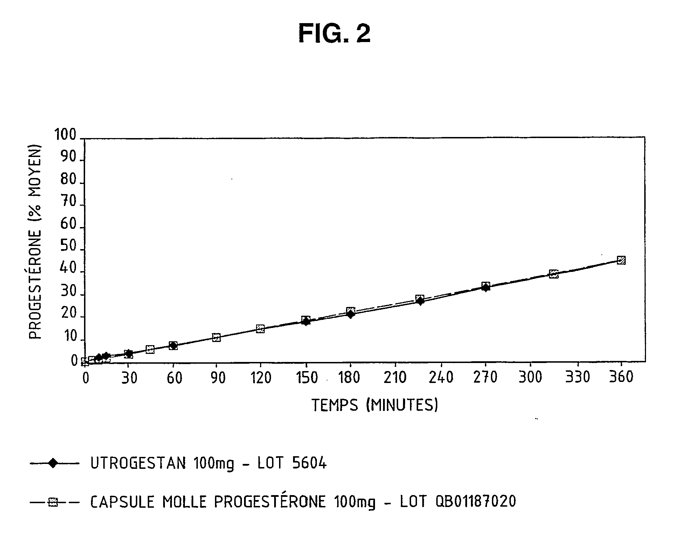

Les résultats reportés à la figure 2 ci-après démontrent que les courbes de dissolution in vitro d'une capsule d'UTROGESTAN® et d'une capsule selon l'invention contenant l'huile de tournesol sont quasi-identiques.

Les caractéristiques physico-chimiques de l'ancienne formulation sont donc conservées dans la formulation selon l'invention.

### EXEMPLE 6 : ETUDE DE BIOEQUIVALENCE ENTRE LES CAPSULES D'UTROGESTAN ET LA COMPOSITION PHARMACEUTIQUE SELON L'INVENTION

Une étude de bioéquivalence a été effectuée afin de comparer des capsules selon l'invention contenant 100 mg de progestérone en suspension dans l'huile de tournesol avec des capsules d'UTROGESTAN®.

L'étude a été effectuée sur un échantillon representatif de 60 femmes, dans des conditions de jeûne.

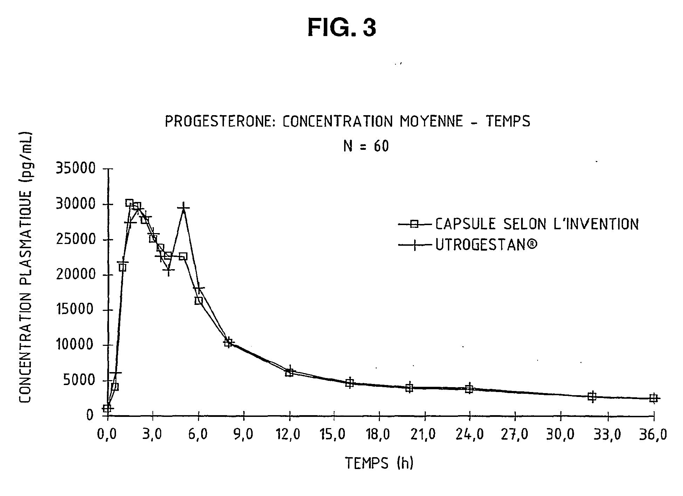

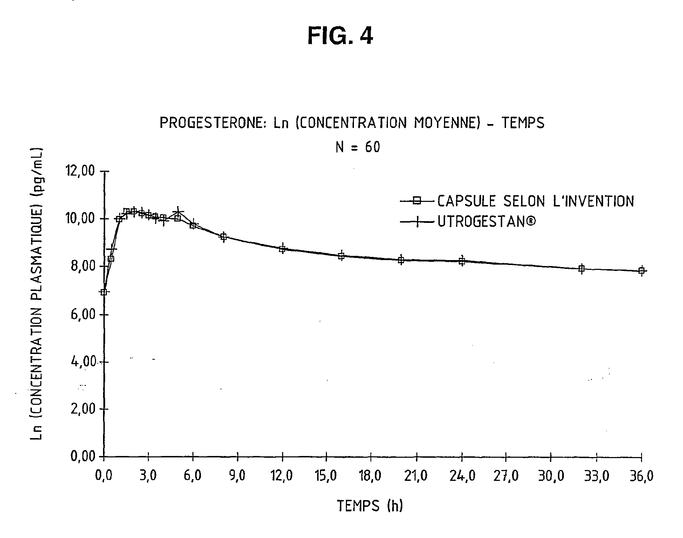

Les résultats de l'étude ont établi une bioéquivalence entre les capsules selon l'invention et UTROGESTAN® (voir Fig. 3 et 4 ci-après).

## Revendications

1. Composition pharmaceutique comprenant de la progestérone micronisée, de la lécithine de soja, et au moins une huile sélectionnée dans le groupe constitué par l'huile de tournesol, d'olive, de sésame, de colza et d'amande.

2. Composition pharmaceutique selon la revendication 1, dans laquelle le rapport progestérone/ huile(s) est compris entre 0,15/1 et 3/1, de préférence entre 0,25/1 et 2/1, préférentiellement entre 0,40/1 et 1/1, et plus préférentiellement encore est de 0,67/1.

3. Composition pharmaceutique selon l'une ou l'autre des revendications 1 et 2, dans laquelle le rapport lécithine de soja/huile(s) est compris entre 0,005/1 et 0,3/1, de préférence entre 0,01/1 et 0,2/1, préférentiellement entre 0,040/1 et 0,1/1, et plus préférentiellement encore est de 0,067/1.

4. Composition pharmaceutique selon l'une quelconque des revendications 1 à 3, comprenant un oestrogène ou un dérivé de type ester, de préférence sélectionné dans le groupe constitué par le 17-β estradiol, l'estrone, le 17α-éthinyl-estradiol, le valérate d'estradiol, ou les phyto-oestrogènes, et plus préférentiellement encore est le 17-β estradiol.

5. Composition pharmaceutique selon l'une quelconque des revendications 1 à 4, dans laquelle au moins la progestérone se trouve en suspension dans la ou les huiles.

6. Composition pharmaceutique selon l'une quelconque des revendications 1 à 5, se présentant sous la forme d'une capsule molle.

7. Spécialité pharmaceutique selon l'une quelconque des revendications 1 à 6, dont chaque unité posologigue comprend entre 2 mg et 600 mg de progestérone micronisée, de préférence entre 30 mg et 300 mg, et plus préférentiellement encore entre 100 mg et 200 mg.

8. Procédé de préparation d'une composition pharmaceutique selon l'une quelconque des revendications 1 à 6, comportant les étapes successives suivantes :
- on réalise, sous agitation, un mélange d'huiles sélectionnées dans le groupe constitué par l'huile de tournesol, l'huile d'olive, de sésame, de colza et d'amande, et de lécithine de soja afin d'obtenir un mélange ;
- on ajoute, sous agitation, la progestérone micronisée au mélange ainsi obtenu afin d'obtenir une suspension homogène.

9. Utilisation de la progestérone micronisée, et de la lécithine de soja, et au moins une huile sélectionnée dans le groupe constitué par l'huile de tournesol, d'olive, de sésame, de colza et d'amande pour la préparation d'un médicament destiné au traitement d'une condition physiologique liée à l'insuffisance de la sécrétion de la progestérone.

10. Utilisation selon la revendication 9, dans laquelle le médicament contient également un oestrogène ou un dérivé de type ester, de préférence sélectionné dans le groupe constitué par le 17-β estradiol, l'estrone, le 17α-éthinyl-estradiol, le valérate d'estradiol, ou les phyto-oestrogènes, et plus préférentiellement encore est le 17-β estradiol.

## Claims

1. Pharmaceutical composition comprising micronized progesterone, soya bean lecithin, and at least one oil selected from the group consisting of sunflower oil, olive oil, sesame oil, colza oil and almond oil.

2. Pharmaceutical composition according to claim 1, in which the progesterone/oil(s) ratio is between 0.15/1 and 3/1, preferably between 0.25/1 and 2/1, preferentially between 0.40/1 and 1/1, and even more preferentially is 0.67/1.

3. Pharmaceutical composition according to any one of claims 1 and 2, wherein the soya bean lecithin/oil(s) ratio is between 0.005/1 and 0.3/1, preferably between 0.01/1 and 0.2/1, preferentially between 0.040/1 and 0.1/1, and even more preferentially is 0.067/1.

4. Pharmaceutical composition according to any one of claims 1 to 3, comprising an oestrogen, or an ester-type derivative thereof, preferably selected from the group consisting of 17-beta-oestradiol, oestrone, 17-alpha-ethinyl oestradiol and oestradiol valerianate, or phyto-oestrogens and even more preferentially is 17-beta-oestradiol.

5. Pharmaceutical composition according to any one of claims 1 to 4, wherein at least the progesterone is in suspension in the oil(s).

6. Pharmaceutical composition according to any one of claims 1 to 5, in the form of a soft capsule.

7. Pharmaceutical product according to any one of claims 1 to 6, wherein each dosage unit comprises between 2 mg and 600 mg of micronized progesterone, preferably between 30 mg and 300 mg, and even more preferentially between 100 mg and 200 mg.

8. Method for preparing a pharmaceutical according to any one of claims 1 to 6, comprising the following successive steps: mixing of oils selected from the group consisting of sunflower oil, olive oil, sesame seed oil, colza oil and almond oil and of soya bean lecithin is carried out, with stirring, in order to obtain a mixture; the micronized progesterone is added, with stirring, to the mixture thus obtained in order to obtain a homogeneous suspension.

9. Use of micronized progesterone, soya bean lecithin, and at least one oil selected from the group consisting of sunflower oil, olive oil, sesame oil, colza oil and almond oil for the preparation of a drug for the treatment of a physiological condition linked to insufficiency of progesterone secretion.

10. Use according to claim 9, in which the pharmaceutical composition further contains an oestrogen, or an ester-type derivative thereof preferably selected from the group consisting of 17-beta-oestradiol, oestrone, 17-alpha-ethinyl oestradiol and oestradiol valerianate, or phyto-oestrogens and even more preferentially is 17-beta-oestradiol.

## Patentansprüche

1. Pharmazeutische Zusammensetzung, umfassend zerkleinertes Progesteron, Sojalecithin, und mindestens ein Öl, das ausgewählt ist aus der Gruppe bestehend aus Sonnenblumen-, Oliven-, Sesam-, Raps- und Mandelöl.

2. Pharmazeutische Zusammensetzung gemäß Anspruch 1, in der das Verhältnis Progesteron/Öl(e) zwischen 0,15/1 und 3/1 liegt, vorzugsweise zwischen 0,25/1 und 2/1, bevorzugt zwischen 0,40/1 und 1/1, und noch weiter bevorzugt bei 0,67/1.

3. Pharmazeutische Zusammensetzung gemäß einem der Ansprüche 1 oder 2, in der das Verhältnis Sojalecithin/Öl(e) zwischen 0,005/1 und 0,3/1 liegt, vorzugsweise zwischen 0,01/1 und 0,2/1, bevorzugt zwischen 0,040/1 und 0,1/1, und noch weiter bevorzugt bei 0,067/1.

4. Pharmazeutische Zusammensetzung gemäß einem der Ansprüche 1 bis 3, umfassend ein Östrogen oder ein Esterderivat, die vorzugsweise ausgewählt sind aus der Gruppe bestehend aus 17-β-Östradiol, Östron, 17α-Ethinyl-östradiol, Östradiolvaleriat oder den Phyto-östrogenen, und noch weiter bevorzugt 17-β-Östradiol ist.

5. Pharmazeutische Zusammensetzung gemäß einem der Ansprüche 1 bis 4, in der zumindest das Progesteron sich in Suspension in dem oder den Ölen befindet.

6. Pharmazeutische Zusammensetzung gemäß einem der Ansprüche 1 bis 5, die in Form einer weichen Kapsel vorliegt.

7. Arzneimittel gemäß einem der Ansprüche 1 bis 6, dessen Dosierungseinheit zwischen 2 mg und 600 mg zerkleinertes Progesteron enthält, vorzugsweise zwischen 30 mg und 300 mg, und noch weiter bevorzugt zwischen 100 mg und 200 mg.

8. Verfahren zur Herstellung einer pharmazeutischen Zusammensetzung gemäß einem der Ansprüche 1 bis 6, das folgende aufeinanderfolgende Schritte umfasst:
- das Erzeugen einer Mischung unter Rühren aus Ölen, die ausgewählt sind aus der Gruppe bestehend aus Sonnenblumenöl, Oliven-, Sesam-, Raps- und Mandelöl, und aus Sojalecithin, um eine Mischung zu erhalten;
- die Zugabe des zerkleinerten Progesterons unter Rühren zur so erhaltenen Mischung, um eine homogene Suspension zu erhalten.

9. Verwendung des zerkleinerten Progesterons, des Sojalecithins und des zumindest einen Öls, das ausgewählt ist aus der Gruppe bestehend aus Sonnenblumen-, Oliven-, Sesam-, Raps- und Mandelöl zur Herstellung eines Medikaments, das zur Behandlung eines physiologischen Zustands bestimmt ist, der mit einer ungenügenden Progesteronsekretion in Zusammenhang steht.

10. Verwendung gemäß Anspruch 9, wobei das Medikament auch ein Östrogen oder ein Esterderivat enthält, die vorzugsweise ausgewählt sind aus der Gruppe bestehend aus 17-β-Östradiol, Östron, 17α-Ethinyl-östradiol, Östradiolvaleriat oder den Phyto-östrogenen, und noch weiter bevorzugt 17-β-Östradiol ist.
